# EUROPEAN PATENT SPECIFICATION

(11) **EP 2 582 359 B1**
(45) Date of publication and mention of the grant of the patent: **12.07.2017**
(21) Application number: 11727518.0
(22) Date of filing: 12.05.2011
(51) Int. Cl.: A61K 45/06, A61K 9/00, A61K 31/375, A23P 10/20, A23P 10/47, A61K 31/122, A61K 31/12

(54) **SOLID COMPOSITIONS IN FORM OF POWDERS OR GRANULATES FOR THE ORAL ADMINISTRATION OF ACTIVE INGREDIENTS AND METHOD FOR OBTAINING THEM**
FESTE ZUSAMMENSETZUNGEN FORM VON PULVERN ODER GRANULATEN FÜR DIE ORALE VERABREICHUNG VON WIRKSTOFFEN UND VERFAHREN ZU IHRER GEWINNUNG
COMPOSITIONS SOLIDES SOUS LA FORME DE POUDRES OU DE GRANULES POUR L'ADMINISTRATION PAR VOIE ORALE DE PRINCIPES ACTIFS ET PROCEDE PERMETTANT DE LES OBTENIR

(30) Priority: 21.06.2010 IT MI20101119
(43) Date of publication of application: 24.04.2013
(73) Proprietor: Labomar S.r.l., 31036 Istrana (TV) (IT)
(72) Inventor: BERTIN, Walter, I-31036 Istrana (TV) (IT); FRATTER, Andrea, I-31036 Istrana (TV) (IT)
(74) Representative: Asensio, Raffaella Consuelo
(86) International application number: PCT/IB2011/001017
(87) International publication number: WO 2011/161501

(56) References cited:
- WO-A1-01/52822
- WO-A1-03/061627
- WO-A1-2006/024237
- WO-A2-2006/085217
- SUGAO H ET AL: "TASTE MASKING OF BITTER DRUG POWDER WITHOUT LOSS OF BIOAVAILABILITYBY HEAT TREATMENT OF WAX-COATED MICROPARTICLES", JOURNAL OF PHARMACEUTICAL SCIENCES, AMERICAN PHARMACEUTICAL ASSOCIATION, WASHINGTON, US, vol. 87, no. 1, 1 January 1998 (1998-01-01), pages 96-100, XP000726388, ISSN: 0022-3549, DOI: DOI:10.1021/JS970104G

## Description

### FIELD OF THE INVENTION

The present invention regards solid compositions in the form of powders or granulates for the oral administration of active ingredients insoluble or poorly soluble in water and/or subjected to degradation phenomena and/or having unpleasant organoleptic properties and/or with low bioavailability and a method for preparing said compositions.

### Background of the invention

Up to date the problem of orally administering active ingredients that are insoluble or poorly soluble in water has been addressed and resolved in various ways.

In particular, patent application ITVE20080055 describes nano-emulsions comprising an aqueous phase containing a base and a lipid phase containing one or more esters of polyoxyethylene sorbitan, wherein the aqueous phase and/or the lipid phase further contains/contain ascorbyl palmitate. The association of esters of polyoxyethylene sorbitan and ascorbyl palmitate salified with emulsifiers allows obtaining highly stable emulsions, suitable for carrying thermolabile or oxidation-sensitive active ingredients, of hydrophilic, lipophilic or amphiphilic type. Such emulsions are prepared with a method which comprises preparing an aqueous phase and a lipid phase, heating said phases at a temperature comprised between 30 and 80°C, preferably between 40 and 70°C, adding the aqueous phase to the lipid phase under mechanical stirring up to the obtainment of a nano-emulsion and quickly cooling the emulsion at ambient temperature. The active ingredients to be vehicled can be added to the aqueous phase or to the lipid phase during the process for preparing the nano-emulsion, or they can be dispersed in the nano-emulsion under mechanical stirring. Furthermore, non-water-soluble powders or vegetable extracts can be moistened using the nano-emulsions to obtain water-dispersible powders or granulates.

However, there are some active ingredients which, besides being insoluble or poorly soluble in water, reveal poor bioavailability if administered orally, due to low absorption or an intense effect of the liver first pass. It would thus be advantageous to have improved compositions capable of vehicling active ingredients even with poor bioavailability when orally administered.

### Description of the invention

Now, it has been discovered that some active ingredients insoluble and/or poorly soluble in water and/or with low bioavailability after oral administration and/or unstable and/or having unpleasant organoleptic characteristics can be formulated in solid compositions in form of powders or granulates constituted by a lipid matrix containing a triglyceride, a polyoxyethylene sorbitan ester and ascorbyl palmitate, and that these formulations can be obtained through a method that does not require any heating and does not imply the preparation of an aqueous phase of ascorbyl palmitate in salified form.

In a first aspect, the invention regards solid compositions in the form of powders or granulates containing:
a) an active ingredient insoluble or poorly soluble in water and/or with low bioavailability after oral administration and/or unstable and/or having unpleasant organoleptic characteristics;
b) a triglyceride;
c) a polyoxyethylene sorbitan ester;
d) ascorbyl palmitate;
e) a polyol and/or a sugar and/or an oligosaccharide and/or un glucosaminoglycan (GAG);
f) an adsorption agent or lipo-gelling-agent.

For the purpose of the present description, the singular expressions "an active ingredient", "a triglyceride", "a sorbitan ester", "a polyol and/or sugar and/or a glucosaminoglycan" and "adsorption agent or lipo-gelling-agent" should be intended to include the corresponding plural expressions as well; therefore, the compositions according to the invention may contain one or more of each of the aforementioned ingredients.

In the compositions according to the invention the ingredients are contained in the following amounts, expressed as percentages by weight with respect to the total weight of the compositions:
one or more active ingredients are contained in amounts comprised between 0.1 and 20%;
one or more triglycerides are contained in amounts comprised between 5 and 30%;
one or more sorbitan esters are contained in amounts comprised between 5 and 30%;
ascorbyl palmitate is contained in an amount comprised between 1 and 7.5%;
one or more polyol/s and/or sugar/s are contained in amounts comprised between 50 and 85%;
the adsorption agent is contained in an amount comprised between 10 and 40%;
one or more absorption agents or lipo-gelling-agents are contained up to the total weight of the composition. According to a preferred embodiment of the invention, the compositions further contain arginine in 1:1 molar ratio with ascorbyl palmitate.

For the purpose of the present description, the expression "an active ingredient insoluble or poorly soluble in water and/or with low bioavailability after oral administration and/or unstable and/or having unpleasant organoleptic characteristics" is used to indicate an active ingredient or a mixture of active ingredients of natural or synthetic origin, which can be obtained through synthesis and semi-synthesis extraction processes, selected from among: polyphenols with molecular weight below 1,000 daltons and insoluble or poorly soluble in water, preferably resveratrol and curcumin (for example in form of dry vegetable extract titrated up to 95% in curcuminoids); omega-3 fatty acids, preferably eicosapentaenoic acid (EPA) and docosahexaenoic (DHA), for example in fish oil variously titrated in EPA and DHA; fat-soluble vitamins; amino acids poorly soluble in water, preferably L-tryptophan; flavonoids; xanthines, preferably caffeine; boswellic acids, preferably 11-beta-keto boswellic acid; essential oils, preferably essential oil of lemon and orange; capsaicin; melatonin; probiotic yeasts, preferably *Lactobacillus rhamnosus* (LGG), *Lactobacillus casei* (LC) and *Lactobacillus acidophilus* (LA). In the previous list, the term flavonoids is used to identify flavones, isoflavones and flavonols, while the expression "lipo-soluble vitamins" is used to identify vitamins A, D, E, F and K.

The expression "insoluble in water" is used to indicate that the solubility is below 1 g on 10000 ml of water; the expression "poorly soluble in water" is used to indicate that 1 g of substance dissolves in a volume of water comprised between 1000 and 10000 ml; the term "triglyceride" is used to identify one or more triglycerides with different carbonaceous length, usually comprised between 6 and 18 carbon atoms, for example coconut oil, wheat germ oil, sunflower seed oil, olive oil, fish oil (EPA and DHA) and triglycerides derived therefrom; triglycerides with average C₈-C₁₀ carbonaceous chain, such as caprylic/capric triglyceride derived from coconut oil are preferably used. Products based on these lipids available on the market are produced by Cognis under the commercial name of Migliol^{®} 810, Migliol^{®} 812, Delios^{®} C, Delios^{®} V, Delios^{®} S or Cetiol^{®} LC. The triglyceride or the mixture of triglycerides may be low or entirely omitted when the active ingredient to be vehicled is a triglyceride.

The term "sorbitan ester" is used to identify one or more sorbitan esters with high HLB, usually comprised between 10 and 20; examples of sorbitan esters with high value of HLB are: polyoxyethylene- (20)-sorbitan monolaurate (polysorbate 20); polyoxyethylene- (20)-sorbitan monopalmitate (polysorbate 40); polyoxyethylene- (20)-sorbitan monostearate (polysorbate 60); polyoxyethylene-(20)-sorbitan tristearate (polysorbate 65); polyoxyethylene- (20)-sorbitan monooleate (polysorbate 80) and polyoxyethylene-sorbitan trioleate (polysorbate 85). According to a preferred embodiment of the invention, the sorbitan ester is selected from among polysorbate 20, polysorbate 60 and polysorbate 80. Preferred examples of polyol are sorbitol and mannitol, glycerine and propylene glycole, while preferred examples of sugar are fructose, sucrose, glucose and trehalose, besides polysaccharides such as maize or rice starch, chitosan with high degree of deacetylation (>90%) and maltodextrins. Examples of GAG are the hyaluronic acid and the chondroitin sulfate.

Examples of absorption agents or lipo-gelling-agents are silica and other silico-aluminates, silica being more preferred.

In a second aspect, the invention regards a method for obtaining solid compositions in the form of powders or granulates for vehicling an active ingredient insoluble or poorly soluble in water and/or unstable and/or having unpleasant organoleptic characteristics comprising the following steps:
a) mixing a triglyceride with a sorbitan ester, ascorbyl palmitate and possibly arginine;
b) adding to the mixture thus obtained the active ingredient and mixing up to the obtainment of a solution, suspension or dispersion;
c) preparing a mixture consisting of a polyol and/or sugar and of an adsorption agent or lipo-gelling-agent and
d) adding the mixture consisting of polyol and/or sugar and adsorption agent or lipo-gelling-agent thus obtained to the solution, suspension or dispersion obtained in step b) and mix up to the obtainment of a powder or flowing granulate and possibly
e) sieving the powder or granulate thus obtained.

The steps a) - e) of the above-indicated method are carried out at ambient temperature, using equipments and methods known to a man skilled in the art.

The amount of each ingredient will be selected from among those described above in the description of the composition. In any case, the amount of polyol and/or sugar and adsorption agent may be suitably adjusted by the man skilled in the art with respect to the volume of the solution or suspension obtained in step a), so as to obtain a granulate or a flowing powder.

The solid compositions according to the invention allow oral administration of active ingredients insoluble or poorly soluble in water masking particularly unpleasant flavours or smells and their method of preparation allows protecting said active ingredients from degradation caused, for example, by heating or oxidizing phenomena. The solid compositions according to the invention are used, mixed with possible excipients or carriers for pharmaceutical or nutritional use, for preparing pharmaceutical or nutraceutical compositions in the form of powders, granulates or tablets. For preparation thereof, possible excipients or carriers can be added together to the polyol and/or sugar and adsorption agent or fat-gelling-agent mixture, or they can be mixed to the powder or granulate obtained in step d) or e), possibly sieving the resulting powder or granulate once again. For the preparation of compositions in form of tablets, the powder or granulate obtained through the aforementioned method are subjected to compression, preferably direct compression, through procedures and equipments known to a man skilled in the art.

The pharmaceutical or nutraceutical compositions according to the invention can be taken up directly given that, in contact with the secretion of the oral cavity or the gastrointestinal tract, form nanodispersions that allow a quick absorption of the active ingredient. The capacity of forming emulsions is particularly marked in the compositions which also contain arginine. Alternatively, the compositions can be dispersed in water and taken up as micro-emulsioned beverages, so as to further increase the bioavailability of the active ingredient, such as for example in the case of omega-3 fatty acids.

Thus, the invention allows overcoming various problems, the one caused by non-solubility in water or by the instability of active ingredients, the one caused by the degradation phenomena, the one caused by unpleasant organoleptic characteristics and the one of improving the bioavailability of lipophilic substrates through sublingual administration. In particular, the method for preparing the compositions according to the invention is particularly advantageous in that it is conducted at ambient temperature, without requiring vigorous mechanical stirring and heating, thus allowing preventing the degradation of thermolabile active ingredients and reducing production costs. Reduction of costs is also obtained due to the fact that the method can be implemented using conventional equipments and mixers for the preparation of solutions and/or dispersions, without requiring using dry or wet granulation techniques.

Now, the invention is illustrated more in detail in the following examples.

### Example 1 - Composition based on 95% curcuma (curcumin)

| *Ingredients* | *grams*/*dosage* |
|---|---|
| CURCUMA 95% | 0.158 |
| DELIOS V (Caprylic/capric triglycerides) | 0.158 |
| VEREMUL T 80 | 0.316 |
| LEMON E.O. | 0.050 |
| ASCORBYL PALMITATE | 0.050 |
| SORBITOL (30-60) | 2.210 |
| AMORPHOUS SILICA | 0.180 |
| SUCRALOSE | 0.015 |
| ORANGE FLAVOUR | 0.200 |
| TOTAL | 3.300 |

### Preparation

First, curcuma at 95%, then, under vigorous stirring, ascorbyl palmitate was added to a mixture of Delios, Veremul and lemon essential oil. The dispersion thus obtained was transferred into a special mixer and sorbitol was added, mixing up to the obtainment of a homogeneous and uniform mixture in granular from, then silica was added, continuing mixing up to obtaining a uniform drying and flowing granulate which was sieved a first time. Sucralose and aroma were mixed in the granulate and the resulting mixture was sieved again. The product was divided into 3 gram single dose packets.

### Composition behaviour after extemporaneous dispersion in water

A packet containing 3.300 grams of composition (corresponding to 158 mg of CURCUMA at 95%) were dispersed in 100 ml of water, stirring with a teaspoon. A translucent dispersion was immediately obtained, without aggregates or yellow-coloured, sweet and aromatic flavour separation oil phases on the surface.

## Claims

1. Solid compositions in the form of powders or granulates containing:
a) an active ingredient selected from among: polyphenols with molecular weight below 1,000 daltons and insoluble or poorly soluble in water, omega-3 fatty acids, fat-soluble vitamins, amino acids poorly soluble in water, flavonoids, xanthines, boswellic acids, essential oils, capsaicin, melatonin, probiotic yeasts and mixtures thereof;
b) a triglyceride;
c) a polyoxyethylene sorbitan ester;
d) ascorbyl palmitate;
e) a polyol and/or sugar and/or a glucosaminoglycan;
f) an adsorption agent and fat-gelling-agent.

2. Compositions according to claim 1 containing arginine in 1:1 molar ratio with ascorbyl palmitate.

3. Compositions according to claim 1 or 2 wherein the active ingredient is selected from among: resveratrol, curcumin, EPA, DHA, vitamins A, D, E, F and K, L-tryptophan, flavones, isoflavones and flavonols; caffeine, 11-beta-keto boswellic acid, essential oil of lemon and orange, capsaicin, melatonin, *Lactobacillus rhamnosus* (LGG), *Lactobacillus casei* (LC), *Lactobacillus acidophilus* (LA) and mixtures thereof.

4. Compositions according to any one of claims 1 to 3 wherein the triglyceride is caprylic/capric triglyceride.

5. Composition according to any one of claims 1 to 4 wherein the sorbitan ester is polysorbate 20, polysorbate 60 or polysorbate 80.

6. Compositions according to any one of claims 1 to 5 wherein the adsorption agent and fat-gelling-agent is silica.

7. Method for preparing solid compositions in the form of powders or granulates containing an active ingredient selected from among polyphenols with molecular weight below 1,000 daltons and insoluble or poorly soluble in water, omega-3 fatty acids, fat-soluble vitamins, amino acids poorly soluble in water, flavonoids, xanthines, boswellic acids, essential oils, capsaicin, melatonin, probiotic yeasts and mixtures thereof, said method comprising the following steps:
a) mixing a triglyceride with a sorbitan ester, ascorbyl palmitate and possibly arginine;
b) adding to the mixture thus obtained the active ingredient and mixing up to the obtainment of a solution, suspension or dispersion;
c) preparing a mixture consisting of a polyol and/or sugar and/or glucosaminoglycan and an adsorption agent and lipo-gelling-agent and
d) adding this mixture to the solution, suspension or dispersion obtained in step b) and mixing up to the obtainment of a powder or flowing granulate and possibly
e) sieving the powder or granulate thus obtained
wherein the steps a) - e) are carried out at ambient temperature.

8. Method according to claim 7 further comprising adding - to the powder or granulate - excipients for pharmaceutical or nutrition use and possibly sieving the resulting powder or granulate.

9. Method according to claim 7 or 8 wherein the active ingredient is selected from among resveratrol, curcumin, EPA, DHA, vitamins A, D, E, F and K, L-tryptophan, flavones, isoflavones, flavonols, caffeine, 11-beta-keto boswellic acid, essential oil of lemon and orange, capsaicin, melatonin, *Lactobacillus rhamnosus* (LGG), *Lactobacillus casei* (LC), *Lactobacillus acidophilus* (LA) and mixtures thereof.

10. Solid compositions in the form of powders or granulates obtainable through the method of any one of claims 7 to 9.

11. Pharmaceutical and/or nutraceutical compositions in form of powders, granulates or tablets containing the solid compositions according to any one of compositions 1) to 6) or 10) mixed with possible excipients or carriers for pharmaceutical or nutritional use.

## Patentansprüche

1. Feststoffzusammensetzungen in Form von Pulvern oder Granulaten, die Folgendes enthalten:
a) einen unter den folgenden ausgewählten Wirkstoff: nicht oder nur schwer in Wasser löslichen Polyphenolen mit einem Molekulargewicht von unter 1.000 Daltons, Omega-3-Fettsäuren, fettlöslichen Vitaminen, schwer in Wasser löslichen Aminosäuren, Flavonoiden, Xanthinen, Boswelliasäure, ätherischen Ölen, Capsaicin, Melatonin, probiotischen Hefen und Mischungen derselben;
b) ein Triglycerid;
c) einen Polyoxyethylensorbitanester;
d) ein Ascorbylpalmitat;
e) einen Polyolalkohol und/oder Zucker und/oder ein Glykosaminoglykan;
f) ein Adsorptionsmittel und Fettgeliermittel.

2. Zusammensetzungen nach Anspruch 1, die Arginin in einem Molarverhältnis von 1:1 mit Ascorbylpalmitat enthalten.

3. Zusammensetzungen nach Anspruch 1 oder 2, bei denen der Wirkstoff unter folgenden ausgewählt wird: Resveratrol, Curcumin, EPA, DHA, Vitamin A, D, E, F und K, L- Tryptophan, Flavonen, Isoflavonen und Flavonolen; Koffein, 11-Keto-ß-Boswelliasäure, ätherischem Zitronen- oder Orangenöl, Capsaicin, Melatonin, Lactobacillus rhamnosus (LGG), Lactobacillus casei (LC), Lactobacillus acidophilus (LA) und Mischungen derselben.

4. Zusammensetzungen nach einem beliebigen der vorangegangenen Ansprüche 1 bis 3, bei dem das Triglycerid Caprylic/Capric Triglyceride ist.

5. Zusammensetzung nach einem beliebigen der Ansprüche 1 bis 4, bei dem der Sorbitanester Polysorbat 20, Polysorbat 60 oder Polysorbat 80 ist.

6. Zusammensetzungen nach einem beliebigen der vorangegangenen Ansprüche 1 bis 5, bei das Adsorptionsmittel und Fett-Geliermittel Kieselsäure ist.

7. Verfahren zur Herstellung von Feststoffzusammensetzungen in Form von Pulvern oder Granulaten, die einen unter nicht oder nur schwer in Wasser löslichen Polyphenolen mit einem Molekulargewicht von unter 1.000 Daltons, Omega-3-Fettsäuren, fettlöslichen Vitaminen, schwer in Wasser löslichen Aminosäuren, Flavonoiden, Xanthinen, Boswelliasäuren, ätherischen Ölen, Capsaicin, Melatonin, probiotischen Hefen und Mischungen derselben ausgewählten Wirkstoff enthalten, wobei das Verfahren die folgenden Schritte umfasst:
a) das Mischen eines Triglycerids mit einem Sorbitanester, Ascorbylpalmitat und möglichst Arginin;
a) das Hinzufügen des Wirkstoffs zu der so erhaltenen Mischung und Mischen bis zum Erhalten einer Lösung, Suspension oder Dispersion;
c) das Herstellen einer Mischung aus einem Polyol und/oder Zucker und/oder Glykosaminglykan und einem Adsorptionsmittel und Fettgeliermittel und
d) das Hinzufügen dieser Mischung zu der unter Schritt b) hergestellten Lösung, Suspension oder Dispersion und Mischen bis zum Erhalten eines Pulvers oder rieselfähigen Granulats und möglichst
e) das Sieben des so erzielten Pulvers oder Granulats, wobei die Schritte a) - e) bei Umgebungstemperatur ausgeführt werden.

8. Verfahren nach Anspruch 7, das weiters das Hinzufügen - zu dem Pulver oder Granulat - von Hilfsstoffen für die pharmazeutische oder Nahrungsmittelverwendung und möglichst das Sieben des sich ergebenden Pulvers oder Granulats umfasst.

9. Verfahren nach Anspruch 7 oder 8, bei denen der Wirkstoff unter Resveratrol, Curcumin, EPA, DHA, Vitamin A, D, E, F und K, L- Tryptophan, Flavonen, Isoflavonen und Flavonolen, Koffein, 11-Keto-ß-Boswelliasäure, ätherischem Zitronen- oder Orangenöl, Capsaicin, Melatonin, Lactobacillus rhamnosus (LGG), Lactobacillus casei (LC), Lactobacillus acidophilus (LA) und Mischungen derselben gewählt wird.

10. Feststoffzusammensetzungen in Form von Pulvern oder Granulaten, die anhand des Verfahrens eines beliebigen der Ansprüche 7 bis 9 erzielbar sind.

11. Pharmazeutische und/oder Nahrungsmittelzusammensetzungen in Form von Pulvern, Granulaten oder Tabletten, die die Feststoffzusammensetzungen nach einer der Zusammensetzungen 1) bis 6) oder 10) gemischt mit möglichen Hilfs- oder Trägerstoffen für pharmazeutische oder Nahrungsmittelverwendungen enthalten.

## Revendications

1. Compositions solides sous forme de poudres ou de granulés contenant :
a) un ingrédient actif sélectionné parmi les suivants : polyphénols possédant un poids moléculaire inférieur à 1 000 daltons et insolubles ou peu solubles dans l'eau, des acides gras oméga-3, des vitamines liposolubles, des acides aminés peu solubles dans l'eau, des flavonoïdes, des xanthines, des acides boswelliques, des huiles essentielles, de la capsaïcine, de la mélatonine, des levures probiotiques et leurs mélanges ;
b) un triglycéride ;
c) un ester de sorbitane polyoxyéthylène ;
d) du palmitate d'ascorbyle ;
e) un polyol et/ou du sucre et/ou un glycosaminoglycanne ;
f) un agent d'adsorption et un agent gras gélifiant.

2. Compositions selon la revendication 1 contenant de l'arginine à une fraction molaire de 1:1 avec du palmitate d'ascorbyle.

3. Compositions selon la revendication 1 ou 2 où l'ingrédient actif est sélectionné parmi les suivants : resvératrol, curcumine, EPA, DHA, vitamines A, D, E, F et K, L-tryptophane, flavones, isoflavones et flavonols ; caféine, acide boswellique 11-béta-cétonique, huile essentielle de citron et d'orange, capsaïcine, mélatonine, Lactobacillus rhamnosus (LGG), Lactobacillus casei (LC), Lactobacillus acidophilus (LA) et leurs mélanges.

4. Compositions selon l'une quelconque des revendications de 1 à 3 où le triglycéride est un triglycéride caprylique/caprique.

5. Composition selon l'une quelconque des revendications de 1 à 4 où l'ester de sorbitane est du polysorbate 20, polysorbate 60 ou polysorbate 80.

6. Compositions selon l'une quelconque des revendications de 1 à 5 où l'agent d'absorption et l'agent gras gélifiant est de la silice.

7. Méthode pour préparer des compositions solides sous forme de poudres ou de granulés contenant un ingrédient actif sélectionné parmi les polyphénols possédant un poids moléculaire inférieur à 1 000 daltons et insolubles ou peu solubles dans l'eau, des acides gras oméga-3, des vitamines liposolubles, des acides aminés peu solubles dans l'eau, des flavonoïdes, des xanthines, des acides boswelliques, des huiles essentielles, de la capsaïcine, de la mélatonine, des levures probiotiques et leurs mélanges, ladite méthode étant composée des étapes suivantes :
a) mélange d'un triglycéride avec un ester de sorbitane, du palmitate d'ascorbyle et le cas échéant de l'arginine ;
b) ajout au mélange ainsi obtenu de l'ingrédient actif et mélange jusqu'à obtention d'une solution, d'une suspension ou d'une dispersion ;
c) préparation d'un mélange composé d'un polyol et/ou de sucre et/ou de glucosaminoglycanne et d'un agent d'absorption et d'un agent gras gélifiant et
d) ajout de ce mélange à la solution, la suspension ou la dispersion obtenue à l'étape b) et mélange jusqu'à obtention d'une poudre ou de granulé coulant et, si possible,
e) tamisage de la poudre ou du granulé ainsi obtenu où les étapes a) à e) sont effectuées à température ambiante.

8. Méthode selon la revendication 7 comprenant en outre l'ajout, à la poudre ou au granulé, d'excipients pour une utilisation pharmaceutique ou nutritionnelle et, le cas échéant, tamisage de la poudre ou du granulé obtenu.

9. Méthode selon la revendication 7 ou 8 où l'ingrédient actif est sélectionné parmi les suivants : resvératrol, curcumine, EPA, DHA, vitamines A, D, E, F et K, L-tryptophane, flavones, isoflavones, flavonols, caféine, acide boswellique 11-béta-cétonique, huile essentielle de citron et d'orange, capsaïcine, mélatonine, Lactobacillus rhamnosus (LGG), Lactobacillus casei (LC), Lactobacillus acidophilus (LA) et leurs mélanges.

10. Compositions solides sous forme de poudres ou de granulés obtenus grâce à la méthode décrite dans l'une quelconque des revendications 7 à 9.

11. Compositions pharmaceutiques et/ou nutritionnelles sous forme de poudres, de granulés ou de tablettes contenant les compositions solides selon l'une quelconque des compositions 1) à 6) ou 10) mélangées avec de possibles excipients ou supports pour une utilisation pharmaceutique ou nutritionnelle.
